# EUROPÄISCHE PATENTSCHRIFT

(11) **EP 1 849 374 B1**
(45) Veröffentlichungstag und Bekanntmachung des Hinweises auf die Patenterteilung: **27.10.2010**
(21) Anmeldenummer: 07003371.7
(22) Anmeldetag: 17.02.2007
(51) Int. Cl.: A42B 3/14

(54) **Kopfgurt**
Headband
Bandeau

(30) Priorität: 28.04.2006 DE 202006007041 U
(43) Veröffentlichungstag der Anmeldung: 31.10.2007
(73) Patentinhaber: AKO-Kunstoffe Alfred Kolb GmbH, 74889 Sinsheim-Hoffenheim (DE)
(72) Erfinder: Zuber, Gerhard, 74909 Meckesheim (DE)
(74) Vertreter: Geitz Truckenmüller Lucht

(56) Entgegenhaltungen:
- US-A- 2 763 006
- US-A- 3 047 876
- US-A- 3 075 201
- US-A- 5 571 217
- US-B1- 6 341 382

## Beschreibung

Die Erfindung betrifft einen Kopfgurt zur gelenkigen Verbindung mit einem Helm, insbesondere einem Schweißerhelm, wobei der Kopfgurt bei bestimmungsgemäßer Benutzung den Kopf des Helmträgers mit einem Stirnband und einem Hinterkopfband umgreift und hierbei die Schädelplatte des Helmträgers von einem Kopfband übergriffen ist, wobei der Kopfgurt dreiteilig aus einem separierbaren Stirnband und einer linken und einer rechten Gurtverzweigung mit je einem Kopfbandstück und je einem Hinterkopfbandstück gebildet ist, die sich bei bestimmungsgemäßer Verbindung zu dem Hinterkopfband und dem Kopfband ergänzen, wobei zusätzlich das Stirnband mit der rechten und linken Gurtverzweigung bestimmungsgemäß verbindbar ist.

Ein solcher Kopfgurt ist, wenn auch vordringlich als Fahrradhelm, aus der DE 200 14 383 U1 vorbekannt. Der Nachteil der mehrteiligen Herstellung des Kopfgurtes wird dabei durch den Vorteil der verbesserten Verstellmöglichkeiten und der Möglichkeit des Einsatzes unterschiedlicher Materialien im Bereich des Kopfgurtes ausgeglichen.

Ein ähnlich mehrteilig aufgebauter Kopfgurt für einen Helm ist auch aus der JP 2004-2 92 978 A bekannt.

EP 0 580 556 A1 offenbart einen einteiligen Kopfgurt, zur gelenkigen Verbindung mit einem Schweißerhelm, mit einem Stirnband, einem Hinterkopfband und einem Kopfband.

Derartige Kopfgurte sind in Verbindung mit Baustellenhelmen, Schweißerhelmen oder auch Fahrradhelmen seit langem bekannt. Bei den meisten Ausführungen ist ein Helm über einen Kopfgurt, der mit vielerlei Einstellmöglichkeiten versehen ist, an die individuelle Kopfform sowie an die individuellen Befindlichkeiten des Benutzers anpassbar. Darüber hinaus besteht üblicherweise eine irgendwie geartete Abstandshalterung zwischen dem unmittelbar mit dem Kopf des Benutzers verbundenen Kopfgurt und dem jeweiligen Helm. Darüber hinaus sind die Helme üblicherweise gelenkig mit dem entsprechenden Kopfgurt verbunden. Der Helm ist also in aller Regel gegenüber dem Kopfgurt verstellbar.

Gegebenenfalls kann der Helm auch in bestimmten Positionen arretiert werden. So ist es aus dem Bereich der Schweißerhelme bekannt, den Helm im Wesentlichen als abklappbares Visier derart auszubilden, dass der Helm entweder das Gesicht gegenüber der Arbeitsstelle, also vorderseitig, vollständig abdeckt oder aber so weit hochgeklappt werden kann, dass das Blickfeld des Benutzers weitgehend freigegeben ist, also das Visier offen ist.

Je nach Größe des zur Herstellung der Kopfgurte eingesetzten Werkzeuges, sind die fraglichen Kopfgurte einstückig oder mehrteilig hergestellt. Bei mehrteiligen Ausführungen sind in aller Regel weitere Verbindungselemente erforderlich, um die Einzelstücke des Kopfgurtes miteinander zu verbinden.

Zur besseren Hautverträglichkeit und zum erhöhten Bedienkomfort ist es darüber hinaus bekannt, den an der Stirn anliegenden Stirnbandbereich zusätzlich zu polstern, mit Lüftungsschlitzen zu versehen oder für das Stirnband ein besonders hautverträgliches, schweißabsorbierendes Material vorzusehen. In aller Regel wird jedoch der Bedien- und Verstellkomfort der Helme als vergleichsweise unbefriedigend empfunden. Auch der Tragekomfort erscheint zumindest für einige Anwendungen verbesserungsbedürftig.

Der Erfindung liegt daher die Aufgabe zugrunde, einen verbesserten Kopfgurt zu schaffen, der insbesondere einen verbesserten Trage- und Bedienkomfort bietet.

Die der Erfindung zugrunde liegende Aufgabe wird durch einen Kopfgurt gemäß Hauptanspruch gelöst. Vorteilhafte Ausgestaltungen der Erfindung ergeben sich gemäß den Merkmalen des Kopfgurtes aus den Ansprüchen 2 bis 18.

Dadurch, dass der erfindungsgemäße Kopfgurt mehrteilig, insbesondere dreiteilig, ausgebildet ist, ergibt sich die Möglichkeit das Stirnband separat herzustellen und insoweit für das Stirnband eher weiche Materialien einzusetzen. Dies ist bei einstückiger Fertigung der fraglichen Kopfgurte nicht möglich, da in anderen Bereichen des Kopfgurtes aus Gründen der sicheren Befestigung oder der Verstellung des Kopfgurtes eher harte Materialien, insbesondere Kunststoffe, gefragt sind. Der mögliche Fertigungsnachteil, dass der Kopfgurt nicht einstückig im Spritzgussverfahren mit einem einzigen Werkzeug herstellbar ist, wird durch den Vorteil eines verbesserten Tragekomforts im Bereich des Stirnbandes mehr als ausgeglichen. Die auch im Übrigen mehrteilige Ausbildung des Kopfgurtes erlaubt es, besser auf die individuellen Bedürfnisse des Benutzers einzugehen, indem im Rahmen der Verbindung der Einzelteile weitere Anpassungen, etwa an die Kopfform des Helmträgers, möglich sind. Außer dem Stirnband umfasst der Kopfgurt zwei Gurtverzweigungen, die je einen Kreuzungsbereich besitzen, der im Wesentlichen die Kreuzungsstelle zwischen dem Kopfbandstück und dem Hinterkopfbandstück der entsprechenden Gutverzweigung darstellt. In diesem Bereich ist eine Gelenklasche aus dem Vollmaterial ausgeschnitten, wobei diese Gelenklasche zur lösbaren Verbindung mit dem Helm dient.

Die lösbare Verbindung von Helm und Kopfgurt erlaubt es von Zeit zu Zeit den Kopfgurt zu wechseln. Insbesondere kann aber der Helm relativ zum Kopfgurt und damit zum Träger individuell nach den jeweiligen Bedürfnissen des jeweiligen Benutzers befestigt werden. Der Helm kann je nach Geschmack relativ zum Kopf des Trägers positioniert werden. Die Gelenklasche kann dabei ganz nach Wunsch als starre oder federnde bzw gedämpfte Verbindung zwischen Helm und Kopfgurt ausgelegt sein.

Dadurch, dass die Gelenklasche aus dem Vollmaterial der Gutverzweigung ausgeschnitten ist, ist diese beweglich gegenüber der übrigen Gurtverzweigung. Dies bietet eine Reihe von weiteren Fertigungsvorteilen, etwa bei der Herstellung der Verbindungselemente zur Verbindung des Kopfgurtes mit dem Helm.

In konkreter und vorteilhafter Ausgestaltung kann die Gelenklasche über einen Federsteg und wenigsten einen Feststellsteg mit der übrigen Gurtverzweigung verbunden sein. In dieser Ausgestaltung ist die Gelenklasche gegenüber dem Übrigen Kopfgurt festgelegt, so dass sich ein weitgehend statischer Helmsitz relativ zum Kopf des Helmträgers ergibt.

In alternativer Ausgestaltung sind die Feststellstege mit wenigstens einer Sollbruchstelle oder zumindest einer Führung für ein Schneidelement, etwa ein Teppichmesser, versehen und können hierdurch bedarfsweise abgetrennt werden. Die Gelenklasche ist nach Abtrennung der Feststellstege nur noch über den bereits erwähnten Federsteg mit dem übrigen Kopfgurt verbunden.

Üblicherweise wird jedoch bereits bei der Herstellung der federnden Helmverbindung bereits auf die Feststellstege verzichtet, also diese erst gar nicht an die Gelenklasche angeformt.

Dies führt jeweils zu einer gelenkig - federnden - Aufhängung des Helms in diesem Bereich. Nachdem bei bestimmungsgemäßer Nutzung der Kreuzungsbereich der Gutverzweigungen ungefähr im Schläfenbereich des Helmträgers anliegt, wird es von vielen Benutzern als angenehm und gegebenenfalls auch als zusätzlicher Sicherungskomfort empfunden, wenn die Verbindung zwischen Helm und Kopfgurt in diesem Bereich federnd bzw. federgedämpft ausgeführt ist. Es gibt auch Benutzer, die gerade diese federnde Verbindung als Unruhemoment empfinden und einen feststehenden Sitz bevorzugen. Die Variante mit den bedarfsweise heraustrennbaren Feststellstegen erlaubt es, mit einem einzigen Helm bzw. mit einer einzigen Ausgestaltung des Kopfgurtes beide Benutzerinteressen zu bedienen.

Die Gelenklasche weist zur Verbindung mit dem Helm eine Ausnehmung auf. Die Verwendung eines Langlochs als Ausnehmung anstelle eines einfachen Stecklochs bietet den Vorteil, dass in diesem Bereich je nach Befestigung des Helms im vorderen oder hinteren Bereich des Langlochs der Helm relativ zum Kopf des Helmträgers weiter vorne oder weiter hinten befestigt werden kann. Üblicherweise wird die Befestigung einmal an die individuellen Interessen des Benutzers, aber auch an die individuelle Kopfform des Benutzers durch die Ausnutzung der durch die beidseits angeordneten Langlöcher gebildeten Verstellmöglichkeiten angepasst. Die Verwendung von Langlöchern stellt also eine zusätzliche Verstellmöglichkeit des Helms relativ zum Kopf des jeweiligen Helmträgers dar.

Grundsätzlich ist selbstverständlich darauf zu achten, dass die Relativpositionierung der Verbindungselemente in den beiden Langlöchern der Gutverzweigung, rechts und links, zumindest annähernd gleich eingestellt wird, weil der Helm sonst schräg oder verdreht sitzt. Es ist daher sinnvoll, den Langlöchern, mithin jeder Gelenklasche auf der dem Federsteg abgewandten Schmalseite der Gelenklasche jeweils eine Markierung zuzuordnen, die es ermöglicht, den Helm auf beiden Seiten des Kopfes auf gleicher Höhe zu befestigen.

Die Gelenklasche als solche ist im Umgebungsbereich des besagten Langlochs mit einer Profilierung versehen, die mit einer entsprechenden Gegenkontur eines Verbindungselementes zur Herstellung der Verbindung mit dem Helm in Eingriff bringbar ist. Die Profilierung sorgt außerdem für eine exakte Positionierung relativ zu den Gurtverzweigungen zu sorgen und darüber hinaus aufgrund der hierdurch gegebenen, stufigen Verstellmöglichkeit sicherzustellen, dass im Bereich beider Gurtverzweigungen zumindest annähernd exakt die gleiche Relativposition des Helms zum Kopf des Benutzers eingestellt wird.

In vorteilhafter Ausgestaltung ist die Profilierung als Wellenprofil mit einer 120° Verzahnung versehen, um einerseits die Relativbeweglichkeit eines mit einer Gegenkontur versehenen Verbindungselementes nicht unnötig zu erschweren und überdies ein erleichtertes Ausrücken des Verbindungselementes von der Gelenklasche weg zu ermöglichen. Wählt man weniger geöffnete Verzahnungen, also etwa eine 90° Verzahnung, so besteht die Gefahr, dass sich die Kunststoffteile der Verbindungselemente plastisch leicht verformen und miteinander so verpresst werden, dass sie nur noch schwer voneinander gelöst werden können. Hierdurch kann im schlimmsten Falle die Verstellmöglichkeit weitgehend verlustigt gehen. Es hat sich daher als vorteilhaft erwiesen, eine offenere Verzahnung, z. B. eine 120° Verzahnung, vorzusehen.

In konkreter Ausgestaltung kann die Verbindung zwischen Kopfgurt und Helm über eine an sich herkömmliche Schrauben/Mutter-Verbindung vermittelt werden. Dabei kann die Verbindung allerdings durchaus unter Zwischenlage weiterer Verbindungselemente erfolgen.

Insbesondere hat es sich als vorteilhaft erwiesen, die Schrauben-/Mutter-Verbindung mit einem selbsthemmenden Gewinde als Aufdrehsicherung zu versehen. Diese kann beispielsweise dadurch realisiert werden, dass mit einer veränderlichen Gewindesteigung gearbeitet wird, so dass, bevor die Verbindung soweit aufgedreht wird, dass die Gefahr besteht, dass die Verbindung sich in ihre Einzelteile zerlegt und anschließend umständlich wieder zusammengesetzt werden muss, eine größere Gewindesteigung vorgesehen ist. Der Benutzer verspürt dann beim Lösen der Verbindung einen gesteigerten Widerstand, der erst überwunden werden muss, um die Verbindung endgültig zu lösen. Hierdurch ist sichergestellt, dass die Schrauben-/Mutter-Verbindung nur gelöst wird, wenn der Benutzer dies tatsächlich will, beispielsweise um einzelne Verbindungselemente auszutauschen. Ein versehentliches Öffnen der Verbindung ist hierdurch weitgehend verhindert.

In alternativer Ausgestaltung kann anstelle einer wechselnden Gewindesteigung auch in dem erwähnten Grenzbereich ein Schlitz oder mehrere Schlitze nachträglich in die Gewinde eingeschnitten werden. Auch die Überwindung eines derart in ein Gewinde eingearbeiteten Schlitzes ist mit zusätzlichem Kraftaufwand verbunden. Auch hier würde die vorstehend beschriebene Hemmwirkung bestimmungsgemäß erreicht.

In abermaliger weiterbildung der Schrauben-/Mutter-Verbindung ist die Verbindungsschraube dieser Verbindung mit einem Kopfstück und einem Gewindeabschnitt versehen, wobei auf der dem Kopfstück zugewandten Seite ein Rundbolzen in die Verbindungsschraube eingearbeitet ist. Dieser Rundbolzen ist wiederum miteinander diametral gegenüberliegenden Kantabschnitten versehen. Diese Kantabschnitte greifen bei bestimmungsgemäßer Montage formschlüssig in entsprechende Ausnehmungen des entsprechend vorgefertigten Helms ein. Die Kantabschnitte dienen somit einer Verdrehsicherung des mit dem Kopfgurt verbundenen Helms.

Als weiteres Element der Schrauben-/Mutter-Verbindung ist auf jeder Seite des Helms je ein Positionierringstück vorgesehen, das auf die Gelenklasche aufgesteckt wird. Dieses Positionierringstück ist auf der der Gelenklasche zugewandten Oberfläche mit der korrespondierenden Gegenkontur zu der Profilierung der Gelenklasche versehen. Selbstverständlich ist auch hierbei eine 120° Verzahnung gegenüber einer scharfkantigeren, also etwa einer 90°, Verzahnung vorzuziehen. Die Gründe hierfür wurden bereits erläutert.

Darüber hinaus an diese Positionierringstücke je ein Klauensteg angeformt. Dieser Klauensteg übergreift die anliegende Gelenklasche und stellt somit zunächst eine Befestigungshilfe für die Verbindungselemente dar. Hierdurch ist nämlich eine erste Halterung des Positionierringstücks relativ zur Gelenklasche gegeben. Darüber hinaus ist über diese, die Gelenklaschen jeweils übergreifenden, Klauenstege sichergestellt, dass beim Lösen der Verbindung die Gelenklasche weiterhin bestimmungsgemäß übergriffen ist, und hierdurch eine Führung bei der Längsbewegung der Verbindungsschraube im Langloch der Gelenklasche gegeben ist und bietet gleichzeitig beim Wiederanziehen der Verbindung eine Verdrehsicherung für die übrigen Verbindungselemente zur Verbindungsschraube.

Als weiteres Verbindungselement ist ein Anschlaghebel vorgesehen, der mit einer Bohrung zur Verbindung mit der vorstehend erwähnten Verbindungsschraube versehen ist. Der fragliche Anschlaghebel besitzt zumindest zwei in Richtung der Gelenklasche vorspringende Anschlagelemente, die die Drehbeweglichkeit der Verbindung gegenüber dem Helm begrenzen. Im Ergebnis stellen die Anschlagelemente auch Drehbegrenzungen für den Helm relativ zum Kopfgurt bzw. zum Kopf des Trägers dar. Über die besagten Anschlagelemente kann zum Beispiel eine Visierfunktion mit wenigstens zwei Endstellungen - Visier offen, Visier zu - realisiert werden.

Beidseits der Ausnehmung zur Ausbildung der Gelenklasche sind mit dem Stirnband korrespondierende Befestigungselemente vorgesehen. Es handelt sich dabei in konkreter Gestaltung um einen Quersteg zur Ausbildung einer Gurtlasche und einen, vorzugsweise verstärkt ausgebildeten, Haltesteg mit entsprechenden Haltelöchern, der mit entsprechenden vorspringenden Verbindungsnoppen des Stirnbandes kraftschlüssig verbunden werden kann.

Nachdem das Stirnband erst nachträglich mit den beiden Gurtverzweigungen verbunden wird, kann es in einem separaten Fertigungsschritt hergestellt werden. Das Stirnband kann daher mit Vorteil aus einem weichen Material hergestellt werden. Im Interesse einer guten und haltbaren Befestigung sowie einer dauerhaften Funktion der Verstellmöglichkeiten des Kopfgurtes sind die übrigen an sensorisch weniger aktiven Bereichen des Kopfes anliegende Elemente des Kopfgurtes aus einem härteren Kunststoff gefertigt.

Das Stirnband weist im Bereich der stirnseitigen Enden des Stirnbandes vorspringende Haltenoppen auf, um bestimmungsgemäß mit den entsprechenden Haltelöchern der Gutverzweigungen verbunden zu werden.

Darüber hinaus ist in das Stirnband im stirnseitigen Endbereich jeweils quer zur Längserstreckung des Stirnbandes ein Rücksprung eingearbeitet, der sich dadurch auszeichnet, dass er gegenüber der übrigen Oberfläche des Stirnbandes zurückspringt. Bei bestimmungsgemäßer Montage des Stirnbandes greift der Quersteg der Gurtverzeigung zumindest annähernd formschlüssig in diesen Rücksprung ein, so dass das Stirnband gemeinsam mit dem Quersteg gegenüber dem Kopf des Benutzers eine, zumindest annähernd, plane Oberfläche ausbildet. Hierdurch werden unangenehme Druckstellen für den Benutzer in diesem Bereich vermieden.

Die beiden Gurtverzweigungen werden nicht allein nur durch das Stirnband miteinander verbunden, sondern auch durch die Kopfbandstücke. Hierzu weisen die Kopfbandstücke einander komplementäre Verbindungselemente auf, die zu dem Kopfband gegebenenfalls unter Verwendung weiterer Zwischenstücke ergänzt werden können.

In konkreter Ausgestaltung sind die Hinterkopfbandstücke jeweils mit wenigstens einem Langloch versehen, das sich in Längsrichtung der Längserstreckung der Hinterkopfbandstücke erstreckt. Bei bestimmungsgemäßer Montage überlappen sich die Hinterkopfbandstücke zumindest im Bereich der Langlöcher derart, dass auch die Langlöcher einander übergreifen, so dass gemeinsame Verbindungselemente die beiden Langlöcher in deren Überlappungsbereich durchgreifen und die Hinterkopfbandstücke hierdurch in diesem Bereich miteinander verbunden sind, so dass die Hinterkopfbandstücke sich auf diese Weise zu dem Hinterkopfband des Kopfgurtes ergänzen.

In vorteilhafter Weiterbildung ist jedes Langloch mit je einer Zahnleiste versehen, wobei ein Langloch eine oberseitige Zahnleiste und das jeweils andere Langloch eine unterseitige Zahnleiste aufweist, so dass sich die beiden Zahnleisten zu einer Verzahnung ergänzen, wobei in diese Verzahnung ein mittels einer Stellschraube betätigbares Zahnrad derart eingreift, dass durch Verdrehung des Zahnrades die beiden Verzahnungen, mithin die Hinterkopfbandstücke entlang ihrer Längserstreckung relativ zueinander verstellbar sind.

Dabei ist dem Zahnrad ein Kraftspeicher - etwa eine Ausrückfeder - derart zugeordnet, dass jeweils erst die Federkraft dieses Kraftspeichers überwunden werden muss, um das Zahnrad mit der aus den beiden Zahnleisten gebildeten Verzahnung in Eingriff zu bringen. Dies hat den Vorteil, dass die Verstellung der Hinterkopfbandstücke nicht versehentlich, sondern nur bewusst betätigt werden kann. Durch die Relativbewegung der Hinterkopfbandstücke zueinander kann der Helm bzw. der Kopfgurt des Helms dem jeweiligen Kopfumfang angepasst werden, also der Helm, je nach Wunsch des Benutzers, mehr oder minder fest befestigt werden. Üblicherweise wird diese Verstellmöglichkeit von jedem Benutzer zu Beginn der Benutzung einmal betätigt. Im Laufe der weiteren Benutzung ist eine weitere Verstellung nur noch ausnahmsweise erforderlich.

Ein weiterer Vorteil der beschriebenen Ausrückfeder besteht darin, dass das Zahnrad mittels der Ausrückfeder außer Eingriff mit den Zahnleisten gebracht wird und in eine Ausrückstellung bewegt wird. In dieser Ausrückstellung ist jedoch der Stellschraube eine Arretierungskontur zugeordnet, die mit der Außenkontur der Stellschraube zusammenwirkt, dass die Stellschraube jeweils in einer definierten Position einhakt, so dass hierdurch eine weitere Sicherung gegen unbeabsichtigtes Verdrehen hergestellt ist.

Die Erfindung wird nachstehend anhand eines in der Zeichnung nur schematisch darstellten Ausführungsbeispiels näher erläutert.

Es zeigen:
- Fig. 1:: eine linke Gurtverzweigung in einer Draufsicht
- Fig. 2:: einen Kreuzungsbereich der linken Gurtverzweigung gemäß Fig. 1 in einer in Fig. 1 bezeichneten Detailansicht, ebenfalls in einer Draufsicht,
- Fig. 3:: den in Fig. 2 gezeigten Kreuzungsbereich in einer Seitenansicht,
- Fig. 4:: den gemäß der Schnittbezeichnung in Fig. 2 gezeigten Kreuzungsbereich in einer Schnittansicht,
- Fig. 5:: eine rechte Gurtverzweigung des Kopfgurtes in einer Draufsicht,
- Fig. 6:: ein Stirnband des Kopfgurtes in einer Draufsicht,
- Fig. 7:: das in Fig. 6 gezeigte Stirnband in einer Seitenansicht,
- Fig. 8:: eine Gurtverzweigung des Kopfgurtes mit angeschlossenen Verbindungselementen zur Verbindung des Kopfgurtes mit einem Helm in einer perspektivischen Zusammenbauskizze,
- Fig. 9 a-c und 9 a'-c :: ein linkes und ein rechtes Positionierringstück der Verbindungselemente, jeweils in einer Rück-, Seiten und perspektivischen Ansicht,
- Fig. 10:: eine Verbindungsschraube der Verbindungselemente in perspektivischer Ansicht,
- Fig. 11:: einen Anschlaghebel der Verbindungselemente in einer Draufsicht und im Querschnitt gemäß der entsprechenden Schnittbezeichnung in dieser Fig. 11,
- Fig. 12:: eine Verbindungsmutter der Verbindungselemente in der Draufsicht und im Querschnitt gemäß der entsprechenden Schnittbezeichnung in dieser Fig. 12 und
- Fig. 13: eine Detaildarstellung zur Verbindung der Hinterkopfbandsstücke in einer perspektivischen Explosionsdarstellung

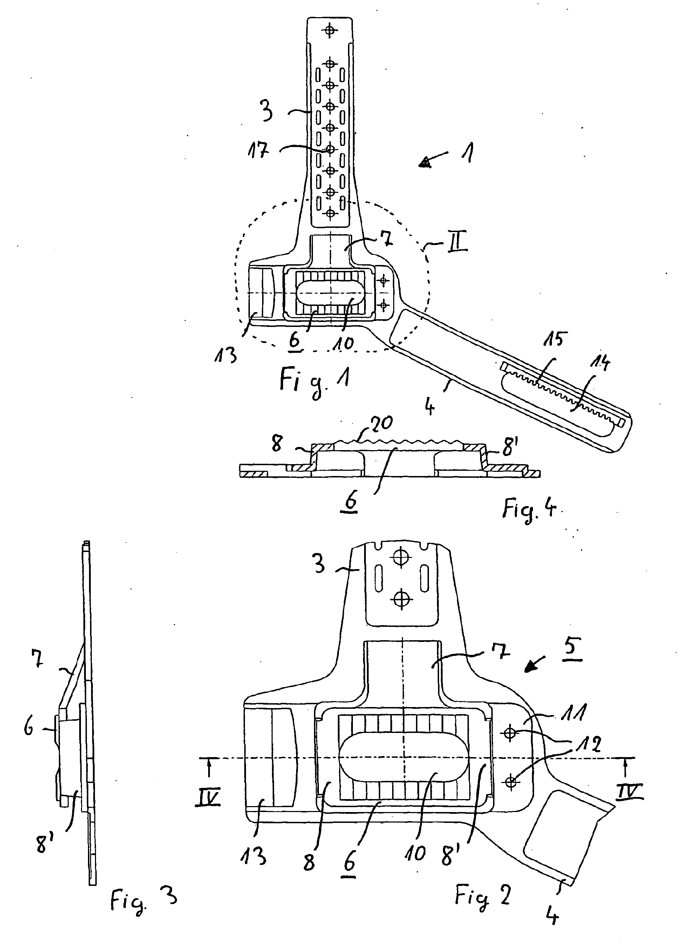

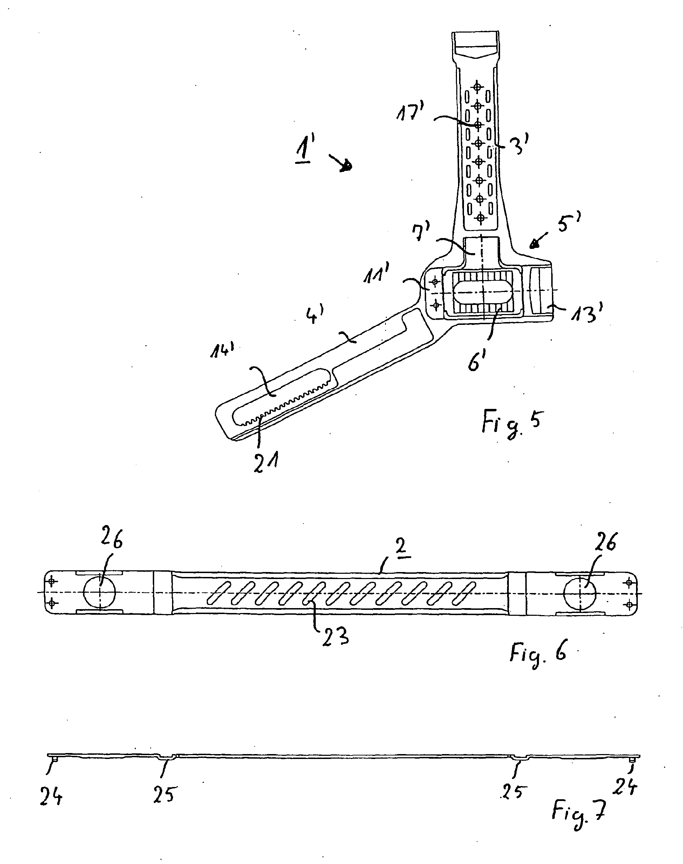

Der in dem Ausführungsbeispiel dargestellte Kopfgurt besteht im Wesentlichen aus einer linken Gurtverzweigung 1 gemäß Figur 1 und einer rechten Gurtverzweigung 1' gemäß Figur 5 sowie einem mit den beiden Gurtverzweigungen 1, 1' zu verbindendes Stirnband 2 gemäß Figur 6.

Nachstehend werden die Einzelteile des Kopfgurtes des Ausführungsbeispiels, wie folgt, beschrieben:
Die in Figur 1 dargestellte linke Gurtverzweigung 1 besteht im Wesentlichen aus einem linken Kopfbandstück 3 und einem linken Hinterkopfbandstück 4, die über einen gemeinsamen Kreuzungsbereich 5 miteinander verbunden sind. Innerhalb dieses Kreuzungsbereiches 5 ist eine Gelenklasche 6 ausgeschnitten, die über einen Federsteg 7 mit der linken Gurtverzweigung 1 verbunden ist. Die Gelenklasche 6 ist beidseits mit je einem Feststellsteg 8, 8' gegenüber dem Hinterkopfbandstück in einem definierten Abstand festgelegt. Nachdem die Gelenklasche 6 die eigentliche Verbindung mit dem Helm ausbildet, ist über die Feststellstege 8, 8' eine feststehende Verbindung zwischen Helm und Kopfgurt ausgebildet. Ggf. kann die Gelenklasche auch ohne die Feststellstege 8,8' oder mir nur einem Feststellsteg 8 oder 8' angeformt sein. Dann ist diese Verbindung eher federnd oder gedämpft ausgelegt.

In konkreter Ausgestaltung der Verbindung zwischen Helm und Kopfgurt ist die Gelenklasche 6 mit einem Langloch 10 versehen. Die Verwendung eines Langlochs 10 anstelle eines Rundbohrung gestattet es, die relative Positionierung des Helm zum Kopfgurt bzw. zum Schädel des Helmträgers in Abhängigkeit von den Umständen des Einzelfalls zu wählen. Beidseits der Gelenklasche 6 sind zusätzliche Befestigungselemente in dem Kreuzungsbereich 5 zur Verbindung der linken Gurtverzweigung 1 mit dem Stirnband 2 vorgesehen. Es handelt sich hierbei um einen vertieft angeordneten Haltesteg 11 mit zwei Haltelöchern 12, in die entsprechende Verbindungsnoppen 14 des Stirnbandes 2 eingeclipst werden können. Auf der anderen Seite der Gelenklasche 6 ist ein Quersteg 13 zur Ausbildung einer Gurtlasche angeformt.

Das an die linke Gurtverzweigung 1 angeformte linke Hinterkopfbandstück 4 ist mit einer Langlochausnehmung 14 versehen. Dieser Langlochausnehmung 24 ist eine Zahnleiste 15 zugeordnet. Auch das linke Kopfbandstück 3 ist mit Verbindungselementen zur Interaktion mit dem rechten Kopfbandstück 3', mit einer geeigneten Lochung 17 versehen.

Die bereits im Zusammenhang mit Fig. 1 geschilderten Details sind in den Fig. 2 und 3 noch einmal ausführlicher dargestellt.

Insbesondere Fig. 4 kann zusätzlich entnommen werden, dass das Langloch 10 der Gelenklasche 6 auf seiner, bei bestimmungsgemäßen Tragen, dem Kopf des Benutzers abgewandten Seite mit einem Wellenprofil 20 versehen ist. Dieses Wellenprofil 20 dient in nachstehend noch näher zu erläuternder Weise einer definierten Festlegung der Verbindungselemente relativ zur Längserstreckung des Langlochs 10 und damit relativ zur Anordnung des Helms gegenüber dem Kopfgurt bzw. dem Kopf des jeweiligen Helmträgers.

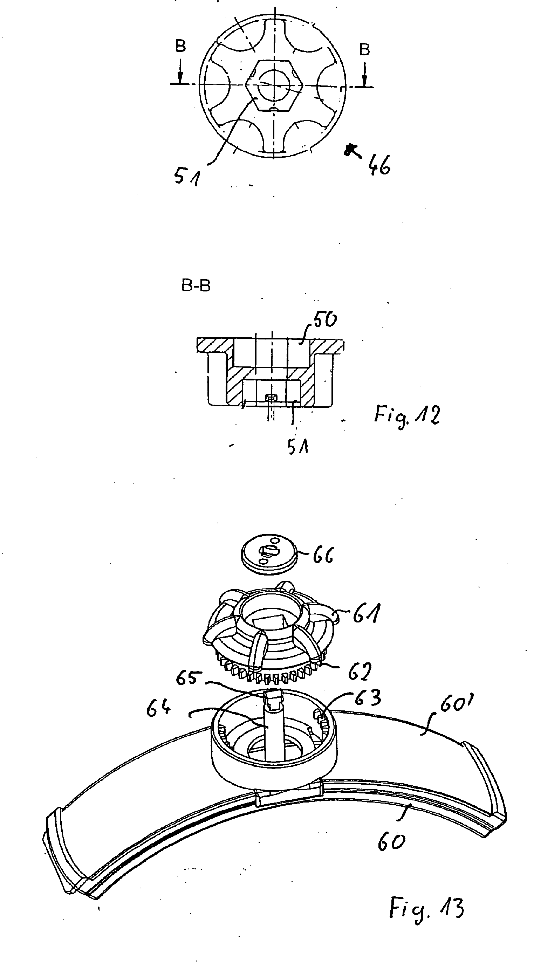

Gemäß der Darstellung in Figur 5 ist auch die rechte Gurtverzweigung 1' analog zur linken Gurtverzweigung 1 ausgebildet. Im Unterschied zur linken Gurtverzweigung 1 weist das rechte Hinterkopfbandstück 4' eine untere Zahnleiste 21 auf, so dass die beiden Zahnleisten 15, 21 der Langlöcher 14, 14' sich zu einer Verzahnung ergänzen, deren genauere Funktion nachstehend in Verbindung mit Fig. 13 noch erläutert wird.

Im Ergebnis ergänzen sich also das linke und das rechte Hinterkopfbandstück 4, 4' zu dem Hinterkopfband, wobei in hier nicht weiter dargestellter Weise sich die beiden Langlöcher der Hinterkopfbandstücke 4, 4' überlappen und hierbei die beiden Zahnleisten 15, 21 eine gemeinsame Verzahnung ausbilden.

Ergänzend ist in Figur 6 das Stirnband 2 in einer Draufsicht dargestellt. Wie aus der Darstellung in Figur 6 zu entnehmen, ist das Stirnband 2 separat von der linken und der rechten Gurtverzweigung 1, 1' zu sehen und kann bedarfsweise mit dieser verbunden werden. Dies gibt zunächst die vorteilhafte Möglichkeit, das Stirnband 2 aus einem besonders weichen Material zu fertigen. Darüber hinaus weist das Stirnband 2 in dem üblicherweise an de Stirn anliegenden Bereich Lüftungsschlitze 23 auf.

Zur Verbindung mit der linken und der rechten Gurtverzweigung 1, 1' ist das Stirnband 2 auf beiden Seiten mit Verbindungsnoppen 24 versehen. Die Verbindungsnoppen 24 können mit den Haltelöchern 12 des Haltestegs 11 der linken und rechten Gurtverzweigung 1, 1' verbunden werden. Dabei greift der Quersteg 13, 13' jeweils in einen ebenfalls beidseits am Stirnband 2 angeformten Rücksprung 25 ein, so dass das Stirnband 2 bei bestimmungsgemäßer Montage eine plane Oberfläche gegenüber dem Kopf des Trägers in diesem Bereich ausbildet.

Darüber hinaus weist das Stirnband in dem Bereich zwischen dem Rücksprung 25 und den Verbindungsnoppen 24 jeweils eine Rundbohrung 26 wodurch die Montage weiterer Verbindungselemente in noch klarzustellender Weise mit der in diesem Bereich jeweils anliegenden Gelenklasche 6, 6' erleichtert ist.

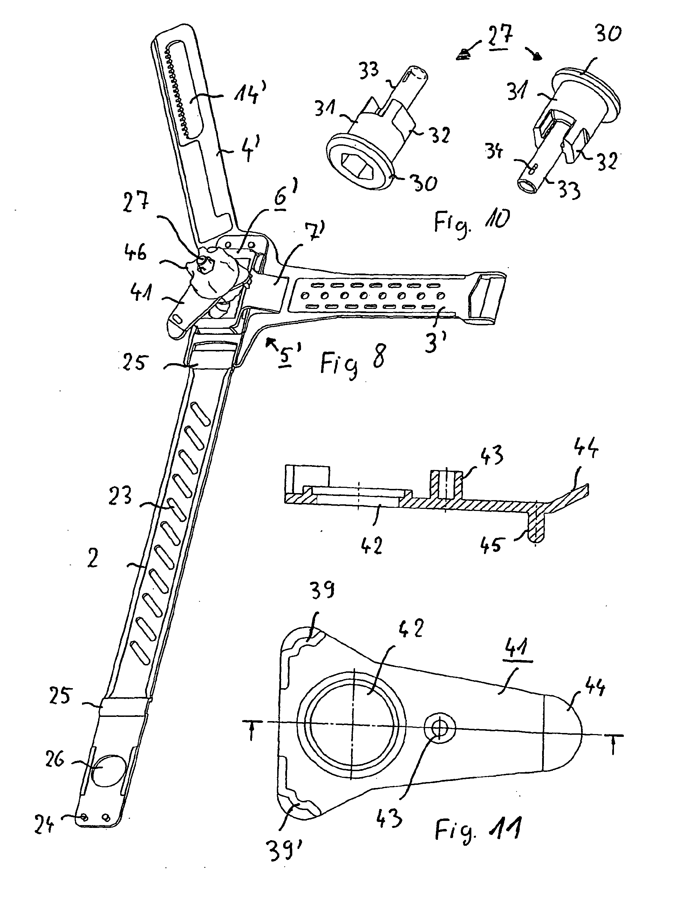

Hierzu ist die Rundbohrung 26 bei bestimmungsgemäßer Montage des Stirnbands 2 in Anlage mit dem Langloch 10 der Gelenklasche 6. Durch die Rundbohrung 26 und das Langloch 10 greifen die weiteren Verbindungselemente, die etwa in Figur 8 im Zusammenbau in perspektivischer Darstellung gezeigt sind.

Wie aus dieser Darstellung ersichtlich, besteht die Verbindung im Wesentlichen aus einer Schrauben-/Mutter-Verbindung, die unter Zwischenlage einer Reihe weiterer Verbindungselemente, zu denen nachstehend noch näher ausgeführt wird, ausgebildet ist.

Gemäß der Darstellung in Figur 8 wird zunächst durch die Rundbohrung 26 des Stirnbands 2 und dann das Langloch 10 der Gelenklasche 6 eine Verbindungsschraube 27 gesteckt. Diese Verbindungsschraube 27 ist in Figur 10 näher dargestellt.

Die Verbindungsschraube 27 besitzt zunächst ein Kopfstück 30, das auf der dem Kopf des Helmträgers zugewandten Seite des Langlochs der jeweiligen Gelenklasche anliegt. An dieses Kopfstück 30 schließt sich ein Rundbolzen 31 der Verbindungsschraube 27 an, der auf der dem Kopfstück 30 abgewandten Seite von zwei einander diametral gegenüberliegenden Kantabschnitten 32 abgeschlossen ist. An diese Kantabschnitte 32 schließt sich ein Gewindeabschnitt 33 an, in den in dem dem Kopfstück 30 abgewandten Bereich diametral einander gegenüberliegende Schlitze 34 zur Unterbrechung des Gewindes eingearbeitet sind. Die Schlitze 34 stellen eine Aufdrehsicherung für die bereits erwähnte Schrauben-/Mutter-Verbindung dar, weil die mit dem Gewindeabschnitt 33 interagierende Verbindungsmutter 46 nur unter Aufbietung zusätzlichen Kraftaufwandes über den Bereich der Schlitze 34 hinaus aufgedreht werden kann.

Die Kantstücke selbst wirken mit einer entsprechenden Mehrkantausnehmung des Helms derart zusammen, dass dieser unter Belassung der gewünschten Verdrehfunktion verdrehsicher festgelegt ist.

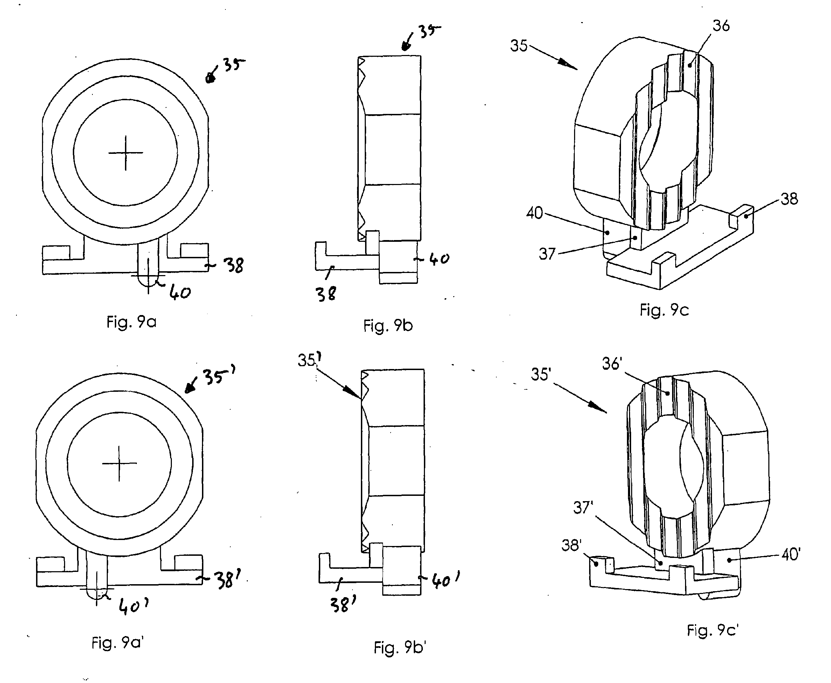

Auf der dem Kopfstück 30 abgewandten Seite der, bei bestimmungsgemäßen Zusammenbau mit der Verbindungsschraube 27 verbundenen, Gelenklaschen 6, 6' ist jeweils ein Positionierringstück 35, 35' auf die Verbindungsschraube 27 aufgesetzt. Dieses Positionierringstücke 35, 35' sind in den Fig. 9 a- c und 9 a'-c' in verschiedenen Ansichten. Die Positionierringstücke 35, 35' weisen jeweils auf der der Gewindelasche 6, 6' zugewandten Oberfläche eine Gegenkontur 36, 36' auf, die mit dem Wellenprofil 20 der Gewindelasche 6 derart zusammenwirkt, dass bei gefestigter, geschlossener Schrauben-/Mutter-Verbindung die Positionierringstücke 35, 35' eindeutig gegenüber der Gelenklasche 6 festgelegt sind. Dann steht die Gegenkonturen 36, 36' jeweils mit dem Wellenprofil 20, 20' in Eingriff. Die Gegenkonturen 36, 36' sind daher wie das Wellenprofil 20, 20' der Gelenklasche 6, 6', jeweils mit einer 120°-Verzahnung versehen, um ein leichteres Ausrücken der Verbindungselemente zu ermöglichen.

Über einen Abstandssteg 37, 37' ist jeweils ein Klauensteg 38 an das Positionierringstück 35, 35' angeformt. Dabei springt der Klauensteg 38 in Richtung der jeweiligen Gelenklasche 6, 6' vor und übergreift diese bei geschlossener Verbindung. An den Abstandssteg 37, 37' ist ein Querstück 40, 40' angeformt. Das Querstück 40 erstreckt sich in seiner Längserstreckung im Wesentlichen parallel zur Verbindungsschraube 27 und dient in noch näher klarzustellender Weise als Anschlag für die Verdrehung eines Anschlaghebels 41, 41', der mit dem Helm verbunden ist. Wie der Vergleich der Fig. 9a und 9a' hinreichend deutlich macht, sind die Positionierringstücke 35 und 35' keine Gleichstücke. Sie unterscheiden sich vielmehr notwendig hinsichtlich der Positionierung des Querstücks 40, 40' relativ zu dem besagten Klauensteg 38, 38' je nachdem ob auf der rechten oder der linken Seite des Helms mit Hilfe des Querstücks 40 ein Anschlag gesetzt werden soll.

Der bereits erwähnte Anschlaghebel 41 ist in Figur 11 in einer Draufsicht und im Querschnitt gezeigt. Der Anschlaghebel 41 besitzt einen zumindest im Wesentlichen dreieckförmigen Querschnitt. Gemäß dieser Darstellung besitzt zunächst auch der Anschlaghebel eine Durchgangsbohrung 42, um auf die Verbindungsschraube 27 gesteckt werden zu können. Auf der dem Positionierringstück 35 zugewandten Seite springen in Richtung des Positionierringstücks 35 zwei Anschlagelemente 39, 39' vor, die mit dem Querstück 40 des Positionierringstücks 35 derart zusammenwirken, dass hierdurch die Drehbeweglichkeit des Anschlaghebels 41 gegenüber dem Positionierringstück 35 begrenzt ist.

Darüber hinaus, springt in Richtung des Positionierringstücks 35 ein weiterer Anschlagpin 43 vor. Je nach Gewicht des Helms kann an der rechten Gurtverzweigung 1' oder an beiden Gurtverzweigungen 1, 1' je ein solcher Anschlaghebel 41 montiert sein. Bei der Montage an der rechten Gurtverzeigung 1' sind das Anschlagelement 39 und der Anschlagpin 43 in Funktion, bei der Montage an der linken Gurtverzweigung 1 dementsprechend der Anschlagpin 43 und das andere Anschlagelement 39'.

Die von der Durchgangsbohrung 42 entfernte Dreiecksseite des Anschlaghebels 41 weist eine Abkantung 44 in Richtung des Positionierringstücks 35 auf. Diese Abkantung 44 stellt ein Griffstück zur Verstellung des Anschlaghebels 41 dar, der ansonsten auf der dem Helm zugewandten Seite, also an der Helminnenseite, plan anliegen würde.

Auf der bei bestimmungsgemäßer Montage dem Positionierringstück 35 abgewandten Oberfläche im Übergangsbereich zur Abkantung 44 ist außerdem ein Verbindungsbolzen 45 angeformt. Der Verbindungsbolzen 45 wird zusätzlich in ein entsprechendes Loch des Helms gesteckt. Bedarfsweise können hierzu in der Helmschale unterschiedliche Löcher vorgesehen sein, um verschiedene Helmpositionen relativ zum Kopf des Trägers zu ermöglichen.

Hieraus folgt wiederum, dass mit der Begrenzung der Drehbeweglichkeit des Anschlaghebels 41 durch die miteinander zusammenwirkenden Anschlagelemente 39, 39'und 43 von Anschlaghebel 41 und der Positionierringstücke 35, 35' die Drehbeweglichkeit des Helms gegenüber dem Kopfgurt und damit dem Helmträger begrenzt ist.

Die zwei Anschläge 39 und 43 können beispielsweise der Stellung - Visier offen, Visier zu - bei einem Schweißerhelm entsprechen.

Geschlossen wird die Verbindung schließlich mit der Verbindungsmutter 46 gemäß der Darstellung in Figur 12. Die Verbindungsmutter 46 schließt die Schrauben-/Mutter-Verbindung mit der Verbindungsschraube 27. Hierzu weist die Verbindungsmutter 46 den Griff einer Flügelmutter auf, die leicht gegriffen und verdreht werden kann. Dabei muss die Verbindungsmutter 46 zunächst über den Gewindeabschnitt 33 mit der Verbindungsschraube 27 verbunden werden. Beim ersten Zudrehen der Verbindung muss dabei Gewindehemmung durch die Schlitze 34 überwunden werden.

Zur Ausbildung der Verbindung von der Verbindungsschraube 27 mit der Verbindungsmutter 46 wird eine Einlegmutter, in einen Innensechskant 51 eingelegt, der zentrisch im Kopf der Verbindungsmutter 46 angeordnet ist. Die Verwendung einer hochwertigen Einlegemutter garantiert eine dauerhaft haltbare und erneuerbare Verbindung.

Auf der der Verbindungsschraube 27 zugewandten Seite der Verbindungsmutter 46 ist eine Bohrung 50 mit einem erweiterten Innendurchmesser vorgesehen, der bei geschlossener Verbindung die Kantabschnitten 32 zentrisch umschließt.

Fig. 13 zeigt die verstellbare Verbindung der Hinterkopfbandstücke 4, 4' die vorliegend von einer Manschettunter- und -oberteil 60, 60' übergriffen sind. Wie bereits erwähnt ergänzen sich die Zahnleisten 15, 21 der Hinterkopfbandstücke 4, 4' zu einer Verzahnung. In diese Verzahnung greift ein mittels einer Stellschraube 61 bewegliches, hier nicht dargestelltes Zahnrad ein. Hierdurch wird eine Relativverstellung der Hinterkopfbandstücke 4, 4' zueinander bewirkt, wobei die Hinterkopfbandstücke 4, 4' hierbei in dem Manschettenunter und -oberteil 60, 60' bestimmungsgemäß geführt sind. Durch die Verstellung der Hinterkopfbandstücke 4, 4' relativ zueinander kann der Helm enger oder weiter gestellt werden.

Um das besagte Zahnrad mit der Verzahnung in Eingriff zu bringen, ist es erforderlich den Widerstand einer in Fig. 13 ebenfalls nicht dargestellten Ausrückfeder zu überwinden. Diese Ausrückfeder bewirkt aber auch dass ein an die Stellschraube 61 angeformtes Zahnrad 62 ausgerückt wird und mit einem konzentrisch zur Stellschraube 61 angeordneten Zahnkranz 63 in Eingriff gelangt, um so die Stellschraube 61 in ihrer jeweiligen Stellung festzulegen.

Um die Stellschraube 61 ihrerseits gegen die Ausrückkraft der Ausrückfeder zu sichern, ist eine Scheibe 66 auf einen Zapfen 64, der an das Manschettenunterteil 60 angeformt ist, aufgesteckt und anschließend mit einer 90°-Drehung arretiert. Die Verdrehung der Scheibe 66 bewirkt, dass eine rechteckige Aussparung in der Scheibe 66, die mit einem Verriegelungssteg 65, der an den Zapfen 64 angeformt ist, korrespondiert, nicht mehr den Zapfen umgreift sondern vielmehr unterhalb des Zapfens gegenüber diesem verdreht angeordnet ist, mithin die Scheibe 66 und damit auch die Stellschraube 61 gesichert ist.

Vorstehend ist somit ein konkretes Ausführungsbeispiel eines aus drei Elementen, nämlich einer linken und einer rechten Gurtverzweigung 1, 1' sowie einem Stirnband 2, zusammensetzbaren Kopfgurt beschrieben, wobei der Kopfgurt eine federnde oder starre Befestigung des Helms an dem Kopfgurt erlaubt. Zusätzlich kann der Helm je nach Relativanordnung der Verbindungselemente in dem Langloch 10 in der Gelenklasche 6 in seiner Position relativ zum Kopf des Helmträgers innerhalb einem großen Verstellbereichs verschoben werden.

Darüber hinaus können über die Anschlagelemente des Anschlaghebels 41 definierte Anschläge im Sinne - Visier offen, Visier zu - ermöglicht sein. Schließlich kann das Hinterkopfband durch die von den beiden Hinterkopfbandstücken 4, 4' ausgebildete Verzahnung mittels einer einfachen Verstellschraube der Kopfform angepasst werden. Auch die Kopfbandstücke können in einfacher Weise zu einem Kopfband ergänzt werden.

Vorstehend ist somit ein neuartiger Kopfgurt beschrieben, der durch einen gesteigerten Bedienkomfort sowie eine Vielzahl von Verstellmöglichkeiten einen bisher nicht da gewesenen Tragekomfort für mit diesem Kopfgurt zu verbindende Helme bietet.

### BEZUGSZEICHENLISTE

- 1,1': linke, rechte Gurtverzweigung
- 2: Stirnband
- 3,3': linkes, rechtes Kopfbandstück
- 4,4': linkes, rechtes Hinterkopfbandstück
- 5,5': linker, rechter-Kreuzungsbereich
- 6,6': linke, rechte Gelenklasche
- 7,7': linker, rechter Federsteg
- 8,8': linker, rechter Feststellsteg
- 10: Langloch
- 11,11': Haltesteg
- 12: Haltelöcher
- 13,13': Quersteg
- 14,14': Langlochausnehmung
- 15: obere Zahnleiste
- 17,17': Lochung
- 20: Wellenprofil
- 21: untere Zahnleiste
- 23: Lüftungsschlitze
- 24: Verbindungsnoppen
- 25: Rücksprung
- 26: Rundbohrung
- 27: Verbindungsschraube
- 30: Kopfstück

- 31: Rundbolzen
- 32: Kantabschnitte
- 33: Gewindeabschnitt
- 34: Schlitz
- 35,35': linkes, rechtes Positionierringstück
- 36,36': Gegenkontur
- 37,37': Abstandssteg
- 38,38': Klauensteg
- 39,39': Anschlagelemente
- 40,40': Querstück
- 41: Anschlaghebel
- 42: Durchgangsbohrung
- 43: Anschlagpin
- 44: Abkantung
- 45: Verbindungsbolzen
- 46: Verbindungsmutter
- 50: erweiterte Bohrung
- 51: Innensechskant
- 60: Manschettenunterteil
- 60': Manschettenoberteil
- 61: Stellschraube
- 62: angeformtes Zahnrad
- 63: Zahnkranz
- 64: Zapfen
- 65: Verriegelungssteg
- 66: Scheibe

## Patentansprüche

1. Kopfgurt zur gelenkigen Verbindung mit einem Helm, insbesondere einem Schweißerhelm, wobei der Kopfgurt bei bestimmungsgemäßer Benutzung den Kopf des Helmträgers mit einem Stirnband (2) und einem Hinterkopfband umgreift und hierbei die Schädelplatte des Helmträgers von einem Kopfband übergriffen ist, **dadurch gekennzeichnet, dass** der Kopfgurt dreiteilig aus einem separierbaren Stirnband (2) und einer linken und einer rechten Gurtverzweigung (1, 1') mit je einem Kopfbandstück (3, 3') und je einem Hinterkopfbandstück (4, 4') gebildet ist, die sich bei bestimmungsgemäßer Verbindung zu dem Hinterkopfband und dem Kopfband ergänzen, wobei zusätzlich das Stirnband (2) mit der rechten und linken Gurtverzweigung (1, 1') bestimmungsgemäß verbindbar ist und aus der linken und der rechten Gurtverzweigung (1, 1'), jeweils in einem Kreuzungsbereich (5, 5') eine Gelenklasche (6, 6') unter Belassung wenigstens eines Federstegs (7, 7') ausgeschnitten und über diese Gelenklaschen (6, 6') der Kopfgurt beidseits mit dem Helm lösbar verbindbar ist, wobei dieser Kreuzungsbereich (5, 5') jeweils zwischen dem Kopfbandstück (3, 3') und dem Hinterkopfbandstück (4, 4') angeordnet ist.

2. Kopfgurt nach Anspruch 1, **dadurch gekennzeichnet, dass** die Gelenklaschen (6, 6') jeweils zusätzlich über wenigstens einen Feststellsteg (8, 8') mit der jeweiligen Gurtverzweigung (1, 1') verbunden ist.

3. Kopfgurt nach Anspruch 2, **dadurch gekennzeichnet, dass** der oder die Feststellsteg/e (8, 8') jeweils mit wenigstens einer Sollbruchstelle zur bedarfsweisen Abtrennung des jeweiligen Feststellstegs (8, 8') von der Gurtverzweigung (1 oder 1') versehen ist/sind.

4. Kopfgurt nach Anspruch 2 oder 3, durch **gekennzeichnet**, dass diese Gelenklaschen (6, 6') zur Verbindung mit dem Helm jeweils eine, vorzugsweise als Langloch (10) ausgebildete, Ausnehmung aufweist.

5. Kopfgurt nach Anspruch 4, **dadurch gekennzeichnet, dass** die Gelenklasche (6, 6') zumindest in den unmittelbar an die Ausnehmung angrenzenden Umgebungsbereich auf der bei bestimmungsgemäßer Benutzung des Kopfgurtes dem Kopf des Helmträgers abgewandten Seite mit einem Profilierung, vorzugsweise mit einem wellenprofil (20) mit einer 120°-Verzahnung, versehen ist, die mit einer entsprechenden Gegenkontur (36) eines Verbindungselements zur Ausbildung der Verbindung mit dem Helm in Eingriff bringbar ist.

6. Kopfgurt nach einem der Ansprüche 5, **dadurch gekennzeichnet, dass** der Helm über je eine Schrauben-/Mutter-Verbindung gegebenenfalls unter Zwischenlage weiterer Verbindungselemente beidseits mit den Gelenklaschen (6, 6') der rechten und linken Gurtverzweigung (1, 1') verbunden ist.

7. Kopfgurt nach Anspruch 6, **dadurch gekennzeichnet, dass** die Verbindungsschrauben (27) der Schrauben-/Mutter-Verbindungen jeweils ein Kopfstück (30) und einen Gewindeabschnitt (33) besitzen, wobei zwischen den Gewindeabschnitt (33) und Kopfstück (30) ein Rundbolzen (31) eingearbeitet ist, in den in dem, dem Gewindeabschnitt (33) zugewandten, Endbereich zwei einander diametral gegenüberliegende Kantabschnitte (32) eingearbeitet sind, die mit einer entsprechend ausgeformten Ausnehmung des Helms formschlüssig in Eingriff bringbar sind.

8. Kopfgurt nach Anspruch 7, **dadurch gekennzeichnet, dass** die Schrauben/Mutter-Verbindungen jeweils unter Zwischenlage eines Anschlaghebels (41) ausgebildet ist, der hierzu mit einer entsprechenden Durchgangsbohrung (42) für die Verbindungsschraube (27) der Schrauben-/Mutter-Verbindung versehen ist und dieser Anschlaghebel (41) wenigstens zwei in Richtung der Gelenklaschen (6, 6') vorspringende Anschlagelemente (39,39',43) aufweist, die jeweils mit einer Außenkontur eines der übrigen Verbindungselemente und/oder der Gelenklaschen (6, 6') derart zusammenwirken, dass die Anschlaghebel (41), mithin der mit den Anschlaghebeln (41, 41') drehfest verbundene Helm jeweils in ihrer Relativposition zum Kopfgurt festgelegt sind.

9. Kopfgurt nach Anspruch 8, **dadurch gekennzeichnet, dass** die Anschlaghebel (41,41') über die Schrauben-/Mutter- . Verbindung unter Zwischenlage eines Positionierringstücks (35, 35') mit der jeweiligen Gelenklasche (6,6') verschraubt sind wobei an die Positionierringstücke (35, 35') jeweils ein mit den Anschlagelementen (39,39',43) des entsprechenden Anschlaghebels (41, 41') zusammenwirkende Außenkontur angeformt ist.

10. Kopfgurt nach einem der vorhergehenden Ansprüche, **dadurch gekennzeichnet, dass** auch die Positionierringstücke (35,35') jeweils mit einer Bohrung zum Durchstecken der Verbindungsschraube (27) der Schrauben/Mutter-Verbindung versehen sind und bei bestimmungsgemäßer Montage im Umgebungsbereich dieser Bohrung eine mit dem Wellenprofil (20) der Gelenklasche (6 oder 6') zusammenwirkende Gegenkontur (36) angeformt ist.

11. Kopfgurt nach einem der vorhergehenden Ansprüche, **dadurch gekennzeichnet, dass** das an die Gurtverzweigungen (1, 1') jeweils zur lösbaren Verbindung mit dem separierbaren Stirnband (2), in einem dem Stirnband (2) zugewandten Anschlussbereich neben dem Ausschnitt für die Gelenklasche (6, 6') jeweils ein Quersteg (13, 13') zur Ausbildung einer Stirnbandlasche angeformt ist und auf der dem Stirnband (2) abgewandten Seite der Gelenklasche (6, 6') jeweils ein, vorzugsweise vertieft angeformter, Haltesteg (11, 11') mit wenigstens einem Halteloch (12) für in Richtung dieses Haltelochs vorspringende korrespondierende Verbindungsnoppen (24) des Stirnbandes (2) angeformt ist.

12. Kopfgurt nach Anspruch 11, **dadurch gekennzeichnet, dass** das Stirnband (2) beidseits in dem bei bestimmungsgemäßer Montage an dem Quersteg (13, 13') der Gurtlasche der Gurtverzweigung (1, 1') anliegenden Bereich jeweils mit einem quer zur Längserstreckung des Stirnbandes (2) verlaufenden Rücksprung 25 versehen ist, die um eine, zumindest annähernd der Stärke des Querstegs (13, 13') entsprechenden Maß gegenüber der übrigen Stirnbandoberfläche zurückspringt.

13. Kopfgurt nach einem der vorhergehenden Ansprüche, **dadurch gekennzeichnet, dass** die Kopfbandstücke (3, 3') der Gurtverzweigung (1, 1') mit einander komplementären Verbindungselementen derart versehen sind, dass die Kopfbandstücke (3), gegebenenfalls unter Einfügung eines Zwischenstücks zu einem Kopfband miteinander verbindbar sind oder die Hinterkopfbandstücke (4, 4') mit einander komplementären Verbindungselementen derart versehen sind, dass die beiden Hinterkopfbandstücke (4, 4') gegebenenfalls unter Einfügung weiterer Zwischenstücke miteinander zur Ausbildung des Hinterkopfbandes verbindbar sind.

14. Kopfgurt nach Anspruch 13, **dadurch gekennzeichnet, dass** die Hinterkopfbandstücke (4, 4') jeweils wenigstens eine Langlochausnehmung (14) aufweisen, deren Längserstreckung jeweils in Längsrichtung der Hinterkopfbandstücke (4, 4') weist, wobei sich die beiden Hinterkopfbandstücke (4, 4') bei bestimmungsgemäßer Montage jeweils zumindest im Bereich der Langlochausnehmung (14, 14') überlappen und in diesem Bereich mittels gemeinsamer Verbindungselemente die beiden einander überlappenden Langlochausnehmungen (14, 14') der beiden Hinterkopfbandstücke (4, 4') durchgriffen sind und diese hierdurch miteinander lösbar zu dem Hinterkopfband ergänzt und verbunden sind.

15. Kopfgurt nach Anspruch 14, **dadurch gekennzeichnet, dass** die Langlochausnehmungen (14, 14') der Hinterkopfbandstücke (4, 4') jeweils entlang einer Längsseite der Langlochausnehmungen (14, 14') mit je einer Zahnleiste (15 oder 21) derart versehen sind, dass die eine Langlochausnehmung (14) des einen Hinterkopfbandstücks (4) eine ober- und die andere Langlochausnehmung (14) des anderen Hinterkopfbandstücks (4') eine unterseitige Zahnleiste (21) aufweist und die gemeinsamen Verbindungselemente ein mittels einer Stellschraube (61) betätigbares Zahnrad umfassen, das bei bestimmungsgemäßer Montage mit einer aus den beiden Zahnleisten (15, 21) gebildeten Verzahnung derart zusammenwirkt, dass durch Drehung dieses Zahnrads die Hinterkopfbandstücke (4, 4') entlang ihrer Längserstreckung relativ zueinander verstellbar sind.

16. Kopfgurt nach Anspruch 15, **dadurch gekennzeichnet, dass** auf das Zahnrad ein Kraftspeicher, vorzugsweise eine Ausrückfeder, einwirkt, deren Federkraft jeweils überwunden werden muss, um das Zahnrad mit der Verzahnung der einander zumindest im Bereich der Langlöcher (10, 14) überlappenden Hinterkopfbandstücke (4, 4') in Eingriff zu bringen.

17. Kopfgurt nach Anspruch 15 oder 16, **dadurch gekennzeichnet, dass** mittels der Ausrückfeder das Zahnrad und die mit diesem drehfest verbundene Stellschraube (61) ausrückbar ist, wobei die Stellschraube (61) mit einem weiteren Zahnrad (62) versehen ist, die in der ausgerückten Arretierungsposition mit einer dieser Stellschraube (61) zugeordneten Zahnkranz (63) in Eingriff gelangt.

18. Kopfgurt nach einem oder mehreren der Ansprüche 15 bis 17, **dadurch gekennzeichnet, dass** die Hinterkopfbandstücke (4, 4') jeweils in dem Bereich zwischen den Langlochausnehmungen (14, 14') und dem Kreuzungsbereich (5, 5') der Gurtverzweigungen (1, 1') mit Führungselementen für das gegebenenfalls in diesem Bereich anliegende, jeweils andere, Hinterkopfbandstück (4',4) versehen sind.

## Claims

1. A head strap for articulated connection with a helmet, especially a welder's helmet, with the head strap, when used as intended, surrounding the head of the helmet wearer with a forehead band (2) and a back-of-the-head band, and the skull cap of the helmet wearer is covered by a headband, **characterized in that** the head strap is formed by three parts made of a detachable forehead band (2) and a left and a right strap branching (1, 1') with one headband piece (3, 3') each and one back-of-the-head band piece (4, 4') each, which, when joined as intended, complement one another to form the back-of-the-head band and the headband, with additionally the forehead band (2) being connectible as intended with the right and the left strap branching (1, 1'), and an articulated tab (6, 6') is cut out from the left and the right strap branching (1, 1') in a crossing zone (5, 5') while leaving a spring crosspiece (7, 7') and the head strap being connectible on both sides with the helmet in a detachable manner via said articulated tabs (6, 6'), with said crossing zone (5, 5') being arranged between the headband piece (3, 3') and the back-of-the-head band piece (4, 4'), respectively.

2. A head strap according to claim 1, **characterized in that** the articulated tabs (6, 6') are each additionally connected via at least one fixing crosspiece (8, 8') with the respective strap branching (1, 1').

3. A head strap according to claim 2, **characterized in that** the fixing crosspiece/fixing crosspieces (8, 8') is/are each provided with at least one predetermined breaking point for severing the respective fixing crosspiece (8, 8') from the strap branching (1 or 1') as required.

4. A head strap according to claim 2 or 3, **characterized in that** said articulated tabs (6, 6') comprise one recess each, preferably arranged as an oblong hole (10), for connection with the helmet.

5. A head strap according to claim 4, **characterized in that** the articulated tab (6, 6') is provided with a profiling, preferably a wave profile (20) with a 120° gearing, at least in the ambient environment directly adjacent to the recess on the side facing away from the head of the helmet wearer when the head strap is used as intended, which gearing can be brought into engagement with a respective counter-contour (36) of a connecting element for forming a connection with the helmet.

6. A head strap according to claim 5, **characterized in that** the helmet is connected via one screw/nut connection each on both sides with the articulated tabs (6, 6') of the right and left strap branching (1, 1'), optionally by interposing further connecting elements.

7. A head strap according to claim 6, **characterized in that** the connection screws (27) of the screw/nut connections each comprise a head piece (30) and a threaded section (33), with a round bolt (31) being incorporated between the threaded section (33) and the headpiece (30), in which round bolt (31) two mutually diametrically opposed edge sections (32) are incorporated in the end region facing the threaded section (33), which edge sections can be brought into interlocking engagement with a respectively shaped recess of the helmet.

8. A head strap according to claim 7, **characterized in that** the screw/nut connections are each formed by interposing a contact lever (41) which is provided for this purpose with a respective passage bore (42) for the connection screw (27) of the screw/nut connection, and said contact lever (41) comprises at least two contact elements (39, 39', 43) protruding in the direction of the articulated tabs (6, 6'), which contact elements each cooperate with the outside contour of one of the remaining connection elements and/or the articulated tabs (6, 6') in such a way that the contact levers (41), and therefore the helmet connected with the contact levers (41, 41') in a torsion-proof manner, are each fixed in their position relative to the head strap.

9. A head strap according to claim 8, **characterized in that** the contact levers (41, 41') are screwed together with the respective articulated tab (6, 6') via the screw/nut connection by interposing a positioning ring piece (35, 35'), with an outside contour cooperating with the contact elements (39, 39', 43) of the respective contact lever (41, 41') being formed on the positioning ring pieces (35, 35').

10. A head strap according to one of the preceding claims, **characterized in that** the positioning ring pieces (35, 35') are also each provided with a bore for passing through the connection screw (27) of the screw/nut connection and a counter-contour (36) which cooperates with the wave profile (20) of the articulated tab (6 or 6') in the case of mounting as intended is formed in the ambient region of said bore.

11. A head strap according to one of the preceding claims, **characterized in that** a crosswise crosspiece (13, 13') for forming the forehead band tab is formed on the strap branchings (1, 1') respectively for a releasable connection with the detachable forehead band (2) in a connection region that faces the forehead band (2) adjacent to the cut-out for the articulated tab (6, 6'), and respectively a holder crosspiece (11, 11'), preferably being formed recessedly, which comprises at least one holder hole (12) for corresponding connection nubs (24) of the forehead band (2) which protrude in the direction of said holding hole is formed on the side of the articulated tab (6, 6') which faces away from the forehead band (2).

12. A head strap according to claim 11, **characterized in that** the forehead band (2) is provided on both sides with a set-back (25) extending transversally to the longitudinal extension of the forehead band (2) in the region resting on the crosswise crosspiece (13, 13') of the strap tab of the strap branching (1, 1') in the case of mounting as intended, which set-back is set back in relation to the remaining forehead band surface by an amount which corresponds at least approximately to the thickness of the crosswise crosspiece (13, 13').

13. A head strap according to one of the preceding claims, **characterized in that** the headband pieces (3, 3') of the strap branching (1, 1') are provided with mutually complementary connection elements in such a way that the headband pieces (3) can be connected with one another to form a headband, optionally by interposing an intermediate element, or the back-of-the-head band pieces (4, 4') are provided with mutually complementary connection elements in such a way that the two back-of-the-head band pieces (4, 4') can be connected with one another to form a back-of-the-head band, optionally by interposing further intermediate elements.

14. A head strap according to claim 13, **characterized in that** the back-of-the-head band pieces (4, 4') each comprise at least one oblong hole recess (14), the longitudinal extension of which respectively faces in the longitudinal direction of the back-of-the-head band pieces (4, 4'), with the two back-of-the-head band pieces (4, 4') overlapping one another at least in the region of the oblong hole recess (14, 14') in the case of mounting as intended, and the two mutually overlapping oblong hole recesses (14, 14') of the two back-of-the-head band pieces (4, 4') are passed through by means of shared connection elements in this region, and said back-of-the-head band pieces thereby being supplemented and connected with each other in a detachable fashion to form the back-of-the-head band.

15. A head strap according to claim 14, **characterized in that** the oblong hole recesses (14, 14') of the back-of-the-head band pieces (4, 4') are each provided with one tooth bar (15 or 21) along one longitudinal side of the oblong hole recesses (14, 14') in such a way that the one oblong hole recess (14) of the one back-of-the-head band piece (4) comprises a tooth bar on the upper side and the other oblong hole recess (14) of the other back-of-the-head band piece (4') has a tooth bar (21) on the bottom side, and the shared connection elements comprise a gearwheel which can be actuated by means of a setting screw (61), which gearwheel cooperates with a gearing formed by both tooth bars (15, 21) in the case of mounting as intended in such a way that by turning said gearwheel the back-of-the-head band pieces (4, 4') are adjustable along their longitudinal extension relatively to one another.

16. A head strap according to claim 15, **characterized in that** a force storage, preferably a release spring, acts upon the gearwheel, the spring force of which respectively must be overcome in order to bring the gearwheel into engagement with the gearing of the back-of-the-head band pieces (4, 4') which overlap one another at least in the region of the oblong holes (10, 14).

17. A head strap according to claim 15 or 16, **characterized in that** the gearwheel and the setting screw (61) which is connected with the same in a torsion-proof manner can be released by means of the release spring, with the setting screw (61) being provided with a further gearwheel (62) which comes into engagement with a gear crown (63) associated with said setting screw (61) in the released locking position.

18. A head strap according to one or several of the claims 15 to 17, **characterized in that** the back-of-the-head band pieces (4, 4') are each provided in the region between the oblong hole recesses (14, 14') and the crossing zone (5, 5') of the strap branchings (1, 1') with guide elements for the respective other back-of-the-head band piece (4', 4) which optionally comes into contact in this area.

## Revendications

1. Courroie de tête destinée à être articulée sur un casque, notamment sur un casque de soudeur, la courroie de tête, utilisée conformément aux prescriptions, enserrant la tête du porteur de casque moyennant un bandeau frontal (2) et une bande occipitale, une bande de tête enjambant ce faisant la carotte crânienne du porteur de casque, **caractérisée en ce que** la courroie de tête est formée de trois parties, à savoir d'un bandeau frontal (2) séparable et d'une branche gauche et d'une branche droite de courroie (1, 1'), chacune ayant un segment de bande de tête (3, 3') et un segment de bande occipitale (4, 4') qui, reliés conformément aux prescriptions, se complètent pour former la bande occipitale et la bande de tête, le bandeau frontal (2) étant de plus apte à être relié conformément aux prescriptions aux branches droite et gauche de courroie (1, 1') et une patte d'articulation (6, 6') étant découpée dans les branches droite et gauche de courroie (1, 1'), respectivement dans une zone de croisement (5, 5'), en laissant au moins une languette formant ressort (7, 7,') et la courroie de tête étant apte à être reliée séparablement au casque des deux côtés par l'intermédiaire de ces pattes d'articulation (6, 6'), cette zone de croisement (5, 5') étant respectivement interposée entre le segment de bande de tête (3, 3') et le segment de bande occipitale (4, 4').

2. Courroie de tête selon la revendication 1, **caractérisée en ce que** les pattes d'articulation (6, 6') sont chacune de plus reliées à la branche de courroie (1, 1') respective par l'intermédiaire d'au moins une languette de fixation (8, 8').

3. Courroie de tête selon la revendication 2, **caractérisée en ce que** la ou les languette(s) de fixation (8, 8') est/sont pourvues d'au moins un point de rupture prévu permettant au besoin de séparer la languette de fixation (8, 8') respective de la branche de courroie (1 ou 1').

4. Courroie de tête selon la revendication 2 ou 3, **caractérisée en ce que** ces pattes d'articulation (6, 6') comportent chacune un évidement de préférence en forme de trou oblong (10) pour permettre la liaison avec le casque.

5. Courroie de tête selon la revendication 4, **caractérisée en ce que** la patte d'articulation (6, 6') est pourvue, du moins dans la zone immédiatement adjacente à l'évidement, sur le côté détourné de la tête du porteur de casque, lorsque la courroie de tête est utilisé conformément aux prescriptions, d'un profilé, de préférence d'un profil ondulé (20) avec une denture de 120° destinée à s'engager dans un contour conjugué (36) correspondant d'un élément de liaison en vue de réaliser la liaison avec le casque.

6. Courroie de tête selon la revendication 5, **caractérisée en ce que** le casque est relié des deux côtés avec les pattes d'articulation (6, 6') des branches droite et gauche de la courroie (1, 1') par le biais d'une liaison vis/écrou respective, le cas échéant avec interposition d'autres éléments de liaison.

7. Courroie de tête selon la revendication 6, **caractérisée en ce que** les vis de liaison (27) des liaisons vis/écrou possèdent chacune une partie tête (30) et une partie filetée (33), un boulon rond (31) étant inséré entre la partie filetée (33) et la partie tête (30), deux arêtes (32) diamétralement opposées et destinées à se mettre en prise par coopération de forme avec un évidement conjugué du casque étant intégrées dans ce boulon rond, dans la partie d'extrémité tournée vers la partie filetée (33).

8. Courroie de tête selon la revendication 7, **caractérisée en ce que** les liaisons vis/écrou sont chacune formées en interposant un levier de butée (41) qui est pourvu à cet effet d'un trou traversant (42) correspondant pour la vis de liaison (27) de la liaison vis/écrou et que ce levier de butée (41) comporte au moins deux éléments de butée (39, 39', 43) faisant saillie en direction des pattes d'articulation (6, 6'), ces éléments de butée coopérant chacun de telle façon avec un contour externe d'un des autres éléments de liaison et/ou des pattes d'articulation (6, 6') que les leviers de butée (41) et par conséquent le casque, solidaire en rotation des leviers de butée (41, 41'), sont fixés chacun dans leur position relative par rapport à la courroie de tête.

9. Courroie de tête selon la revendication 8, **caractérisée en ce que** les leviers de butée (41, 41') sont vissés sur la patte d'articulation (6, 6') respective par l'intermédiaire de la liaison vis/écrou avec interposition d'un élément d'anneau de positionnement (35, 35'), un contour externe coopérant avec les éléments de butée (39, 39', 43) du levier de butée (41, 41') correspondant étant venu de moulage avec les anneaux de positionnement (35, 35') respectifs.

10. Courroie de tête selon l'une des revendications précédentes, **caractérisée en ce que** les éléments d'anneau de positionnement (35, 35') sont eux aussi pourvus chacun d'un trou pour y passer la vis de liaison (27) de la liaison vis/écrou et qu'un contour conjugué (36) coopérant avec le profil ondulé (20) de la patte d'articulation (6 ou 6') est venu de moulage dans la zone ambiante de ce trou lorsque le montage se fait conformément aux prescriptions.

11. Courroie de tête selon l'une des revendications précédentes, **caractérisée en ce qu'**une languette transversale (13, 13') destinée à former une patte pour le bandeau frontal est venue de moulage sur les branches de courroie (1, 1') respectivement en vue de réaliser une liaison amovible avec le bandeau frontal (2) séparable dans une zone de raccordement tournée vers le bandeau frontal (2) à côté de la découpe pour la patte d'articulation (6, 6') et qu'une languette de maintien (11, 11') venue de moulage, de préférence plus en profondeur, avec au moins un trou de maintien (12) pour des picots de liaison (24) du bandeau frontal (2) correspondants faisant saillie en direction de ce trou de maintien est venue de moulage sur le côté de la patte d'articulation (6, 6') qui est détourné du bandeau frontal (2).

12. Courroie de tête selon la revendication 11, **caractérisée en ce que** le bandeau frontal (2) est pourvu des deux côtés, dans la zone adjacente à la languette transversale (13, 13') de la patte de courroie des branches de courroie (1, 1'), en cas de montage conforme aux prescriptions, d'un décrochement (25) s'étendant transversalement par rapport au bandeau frontal (2) dans le sens de sa longueur, ce décrochement ayant, par rapport à la surface du bandeau frontal, un retrait correspondant du moins approximativement à l'épaisseur de la languette transversale (13, 13').

13. Courroie de tête selon l'une des revendications précédentes, **caractérisée en ce que** les segments de bande de tête (3, 3') des branches de courroie (1, 1') sont pourvus d'éléments de liaison mutuellement complémentaires de telle sorte que les segments de bande de tête (3) sont aptes à être reliés ensemble, le cas échéant en insérant une pièce intercalaire, de manière à former une bande de tête ou que les segments de bande occipitale (4, 4') sont pourvus d'éléments de liaison mutuellement complémentaires de telle sorte que les deux segments de bande occipitale (4, 4') sont aptes à être reliés pour former la bande occipitale, le cas échéant moyennant l'insertion de pièces intercalaires supplémentaires.

14. Courroie de tête selon la revendication 13, **caractérisée en ce que** les segments de bande occipitale (4, 4') comportent chacun du moins un évidement en forme de trou oblong (14), ces évidements s'étendant chacun, dans le sens de leur longueur, suivant la direction longitudinale des segments de bande occipitale (4, 4'), les deux segments de bande occipitale (4, 4') se chevauchant du moins dans la zone de l'évidement sous forme de trou oblong (14, 14') en cas de montage conforme aux prescriptions, et des éléments de liaison communs traversant dans cette zone les deux évidements sous forme de trou oblong (14, 14') se chevauchant des deux segments de bande occipitale (4, 4') de sorte que ceux-ci sont assemblés et reliés de façon amovible pour former la bande occipitale.

15. Courroie de tête selon la revendication 14, **caractérisée en ce que** les évidements sous forme de trou oblong (14, 14') des segments de bande occipitale (4, 4') sont pourvus chacun le long d'un long côté des évidements sous forme de trou oblong (14, 14') d'une crémaillère (15 ou 21) respective de telle sorte que l'un des évidements sous forme de trou oblong (14) de l'un des segments de bande occipitale (4) comporte une crémaillère située sur la face supérieure et que l'autre des évidements sous forme de trou oblong (14) de l'autre des segments de bande occipitale (4') comporte une crémaillère (21) située sur la face inférieure et que les éléments de liaison communs comprennent une roue dentée apte à être actionnée par une vis de réglage (61), cette roue dentée coopérant de telle sorte avec une denture formée des deux crémaillères (15, 21) en cas de montage conforme aux prescriptions que les segments de bande occipitale (4, 4') sont réglables relativement les uns par rapport aux autres dans le sens de leur longueur en faisant tourner cette roue dentée.

16. Courroie de tête selon la revendication 15, **caractérisée en ce qu'**un mécanisme à ressort, de préférence un ressort de désengrènement, agit sur la roue dentée, la raideur de ce ressort devant être surmontée pour permettre à la roue dentée de se mettre en prise avec la denture des segments de bande occipitale (4, 4') se chevauchant, du moins dans la zone des trous oblongs (10, 14).

17. Courroie de tête selon la revendication 15 ou 16, **caractérisée en ce que** la roue dentée et la vis de réglage (61) solidaire en rotation de celle-ci sont aptes à être désengrenées au moyen du ressort de désengrènement, la vis de réglage (61) étant pourvue d'une roue dentée (62) supplémentaire qui se met en prise avec une couronne dentée (63) associée à cette vis de réglage (61) dans la position d'arrêt désengrenée.

18. Courroie de tête selon une ou plusieurs des revendications 15 à 17, **caractérisée en ce que** les segments de bande occipitale (4, 4') sont pourvus d'éléments de guidage pour l'autre segment de bande occipitale (4, 4') respectif, le cas échéant contigu à cette zone, respectivement dans la zone située entre les évidements sous forme de trou oblong (14, 14') et la zone de croisement (5, 5') des branches de courroie (1, 1').
